# EUROPEAN PATENT APPLICATION

(11) **EP 0 843 008 A1**
(43) Date of publication of application: **20.05.1998**
(21) Application number: 96118297.9
(22) Date of filing: 14.11.1996
(51) Int. Cl.: C12N 15/12, C12Q 1/68, C07K 14/47, C12Q 1/00

(54) **Methods for producing the Anaphase Promoting Complex**

(71) Applicant: BOEHRINGER INGELHEIM INTERNATIONAL GmbH, 55218 Ingelheim am Rhein (DE)
(72) Inventor: Nasmyth, Kim, 1010 Wien (AT); Zachariae, Wolfgang, 1190 Wien (AT); Galova, Marta, 1040 Wien (AT)

(57) **Abstract**

Methods for identifying novel subunits of the human Anaphase Promoting Complex (APC). The subunits may be identified first in yeast cells or directly in human cells and then be used for producing recombinant human APC. Use of recombinant APC in a screening assay to find substances that inhibit rapidly proliferating cells by interfering with the cells' entry into the subsequent cell cycle.

## Description

The invention relates to the regulation of the cell cycle in eucaryotic cells.

The aim of most chemotherapeutic approaches against cancer is to kill rapidly proliferating cells while leaving non-proliferating, differentiated cells unaffected. Since the state of the components regulating the cell cycle is different between proliferating and quiescent cells, these components are potential targets for anti-cancer drugs.

The invention relates to the use of a recently identified key regulator of the cell cycle, the Anaphase Promoting Complex or cyclosome, as a target for chemotherapeutic drugs.

It has been shown that mitotic cyclin degradation is required for the final exit from mitosis (Murray et al., 1989; Surana et al., 1993) and is a prerequisite for S-phase in the subsequent cell cycle (Dahman et al., 1995). In extracts from *Xenopus* eggs, degradation of cyclin B depends on a particle called the Anaphase Promoting Complex (APC), which contains at least eight different proteins. The APC and the cyclosome, a particle found in clam oocytes, function as cell cycle regulated ubiquitin-protein ligases that mediate destruction box-dependent ubiquitination (King et al., 1995; Sudakin et al., 1995) and thereby target cyclins for proteolysis by the proteasome (Ciechanover, 1994; Peters, 1994). In the yeast *Saccharomyces cerevisiae*, the isolation of mutants defective in cyclin degradation led to the identification of the tetratricopetide repeat (TPR) proteins Cdc16p, Cdc23p, and Cdc27p as subunits of the APC (King et al., 1995; Irniger et al, 1995; Zachariae and Nasmyth, 1996). These proteins are required for the onset of anaphase in various organisms (Hirano et al., 1988; O'Donnell et al., 1991; Samejima and Yanagida, 1994; Tugendreich et al., 1995; Lamb et al., 1994). Because cyclin proteolysis per se is not required for anaphase (Surana et al., 1993; Holloway et al., 1993), it has been suggested that the APC also targets for destruction proteins whose degradation is necessary for sister chromatid separation (Irniger et al., 1995; Funabiki et al., 1996).

There two main reasons why the APC fulfils the requirements of a target for chemotherapeutic drugs:
1. The activity of the APC is essential for sister chromatid separation and function of the mitotic spindle during cell poliferation. Interfering with its function would prevent tumor cells from completing mitosis.
2. Most tumor cells have highly abnormal karyotypes. They undergo anaphase in the presence of chromosomal damage that would prevent activation of the APC in normal cells. Tumor cells might therefore be especially sensitive to drugs that interfere with APC function.

The identification of all the subunits of the APC is a prerequisite for the understanding of the function and regulation of this key regulator of the cell cycle. In addition, the identification of the APC subunits provides a basis for interfering with the function of APC.

In the experiments of the present invention, two further subunits of the APC of the budding yeast *Saccharomyces cerevisiae* have been identified. It was further established that the yeast APC is a 36S particle that contains at least seven different proteins including the previously identified TPR proteins Cdc16p, Cdc23p, and Cdc27p. The first newly identified subunit is the largest subunit of the yeast APC. The sequence of *APC1* corresponds to the open reading flame YNL172W on chromosome XIV which was determined during the sequencing of the entire genome of *Saccharomyces cerevisiae*. The *APC1* sequence is currently available in the *Saccharomyces* Genome Database. In the experiments of the present invention, *APC1* was shown to be an essential gene. The Apc1 protein shows similarity to BIMEp from *Aspergillus nidulans* (Engle et al., 1990) and to the murine *tsg24* gene product (Starborg et al., 1994). The largest subunit of the APC therefore appears to be conserved between fungi and animal cells.

The second newly identified subunit is encoded by the *CDC26* gene (Akari et al., 1992). In the experiments of the present invention *CDC26* was shown to be essential for APC activity at elevated temperatures.

Human homologs of the yeast Cdc16 and Cdc27 proteins have been identified (Tugendreich et al. 1993; Tugendreich et al., 1995). They are shown to be subunits of a large complex. It can therefore be assumed that human homologs also exist for the remaining, as yet unidentified, subunits of the yeast APC. This notion is supported by the identification of a murine homolog (the *tsg24* gene product) of the yeast Apc1 protein.

The findings of the present invention are therefore highly relevant for human cells and provide a basis for the identification of the subunits of the human APC. The identification of the human subunits is a prerequisite for establishing screening methods designed to find drugs that interfere with the function of the human APC. These drugs have the potential to inhibit rapidly poliferation cells such as tumor cells.

An efficient way to find substances that affect the activity of the APC is an assay that is capable of screening large numbers of compounds. To set up a suitable screening system for identifying such compounds, a source which provides large amounts of APC with a highly reproducible activity is required. Recombinant human APC is considered as the best source for such an assay.

In a first aspect, the present invention provides methods for the identification of the subunits of the human APC by first identifying the corresponding yeast subunits.

This method for identifying novel subunits of the human APC is characterized by the steps
a) replacing in a cells of the budding yeast *Saccharomyces cerevisiae* one or more endogenous genes encoding a known APC subunit by epitope-tagged versions of said genes or transforming the cell with a vector containing the corresponding epitope-tagged cDNA(s),
b) growing cells obtained in a) and preparing a protein extract,
c) isolating the APC by contacting the protein extract obtained in b) with one or more antibodies directed against the epitope-tag(s),
d) isolating the antibody-bound protein(s) and purifying them,
e) determining the sequence of the protein(s),
f) identifying the human subunit(s) by comparing the sequence(s) of the yeast protein(s) obtained in e) and/or the DNA sequence encoding that protein with published human sequences.

According to this method, the APC subunits are first identified in yeast. The yeast subunits are identified by purification of the APC particle from yeast. One or more genes encoding a known subunits (e.g. CDC16) are replaced by an epitope-tagged version (e.g. CDC16-myc6) and the APC is isolated from a protein extract prepared from such a strain. The essential part of the purification procedure is an immuno-affinity purification using one or more antibodies directed against the epitope tag(s) (e.g. the monoclonal antibody 9E10 directed against the myc epitope). Isogenic strains lacking the epitope tag are used as negative controls. Bound protein is separated by gel electrophoresis and subunits are identified by protein micro-sequencing or mass-spectrometry. Once the yeast subunits have been identified, the human homologues can be found by comparison with published sequences, e.g. by searching data bases. This method of the invention takes advantage of the fact that the genome of the budding yeast *Saccharomyces cerevisiae* has been fully sequenced. Therefore amino acid sequences obtained from the isolated subunits allow for direct identification of the respective gene.

According to an alternative method of the invention the human APC or its subunits, respectively, can be directly isolated from human cells by a method related to the one described above.

This method for identifying novel subunits of the human anaphase promoting complex (APC) is characterized by the steps
a) replacing in a human cell one or more endogenous genes encoding known APC subunits by epitope-tagged versions of said genes or transforming the cell with a vector containing the corresponding epitope-tagged cDNA(s) and establishing a cell line,
b) growing the cell line obtained in a) and preparing a protein extract,
c) isolating the APC by contacting the protein extract obtained in b) with one or more antibodies directed against the epitope-tag(s),
d) isolating the antibody-bound protein(s) and purifying them,
e) determining the sequence of the protein(s).

The human cell line is established to express one or more epitope-tagged known human APC subunits such as CDC16Hs or CDC27Hs (Tugendreich et al., 1993; Tugendreich et al., 1995). For transient expression, the cells are transformed with a suitable vector containing the cDNA sequences encoding the known subunit(s). The APC is purified from an extract prepared from such a cell line by immuno-affinity purification using one or more antibodies against the epitope tags. Amino acid sequences determined from the purified proteins are used to search data bases. Alternatively, oligonucleotides may be designed that are based on the determined amino acid sequences and used as probes to screen cDNA libraries.

There is no limitation as to the use of epitope sequences for tagging the proteins; suitable epitope tags are commercially available or may be prepared according to standard methods; an example for methods for the construction of yeast strains expressing epitope-tagged APC subunits is described in Example 4A. If more than one subunits are tagged, the same or, preferably, different epitopes may be used for tagging The purification of the APC by immunoprecipition can be carried out by standard methods, an example with the anti-myc antibody from strains expressing myc-tagged APC-subunits is described in Example 4C. For preparative purposes APC may be isolated from larger, unlabeled cultures, and proteins may be detected on gels by staining with silver or Coomassie Blue rather than autoradiography.

In a related aspect, the present invention further provides methods for producing functional APC complexes assembled from recombinant APC subunits.

Once the APC subunits have been identified and cDNA clones have been isolated, active complexes are generated using recombinant subunits.

In order to obtain the recombinant subunits, the cDNAs encoding the yeast or the human subunits, respectively, can be expressed, together or separately, using established expression systems such as Baculovirus or the yeast Pichia pastoris, which are well known in the art (O'Reilly et al., 1992) and commercially available, e.g. the Pichia pastoris system by Invitrogen. Both of these expression systems offer the possibility to simultaneously express several subunits to generate active complexes.

Unless otherwise stated, the individual steps of the methods of the invention, e.g. epitope-tagging, transformation of yeast strains or human cells, purification procedures, immunoreactions, cloning and expression, may be carried out according to standard protocols that are described in laboratory manuals, e.g. by Ausubel, et al., 1994; Harlow and Lane, 1988; Guthrie and Fink, 1991.

To determine whether the obtained APC complexes are functional and/or whether all the subunits are required for the APC to be functional, the preparation obtained by assembling the recombinantly produced subunits is tested for its ability to ubiquitinate known APC substrates such as cyclin B. An example for a suitable assay is described by Zachariae and Nasmyth, 1996.

Once active complexes have been generated, they can be used to screen for substances that interfere with the activity of the APC.

In an embodiment of the invention, the subunits that are used to form a functional APC are derived from yeast APC.

In a preferred embodiment of the invention, the subunits that are used to form a functional APC are derived from human APC.

In a related aspect the present invention relates to methods for screening substances that interfere with the cell's entry into the subsequent cell cycle and thus inhibit rapidly proliferating cells, i.e. tumor cells. The methods of the present invention are based on the physiological role of the APC to function as a ubiquitin-protein ligase for B-type cyclins and thus make them susceptible to degradation, which is a requirement for the onset of anaphase.

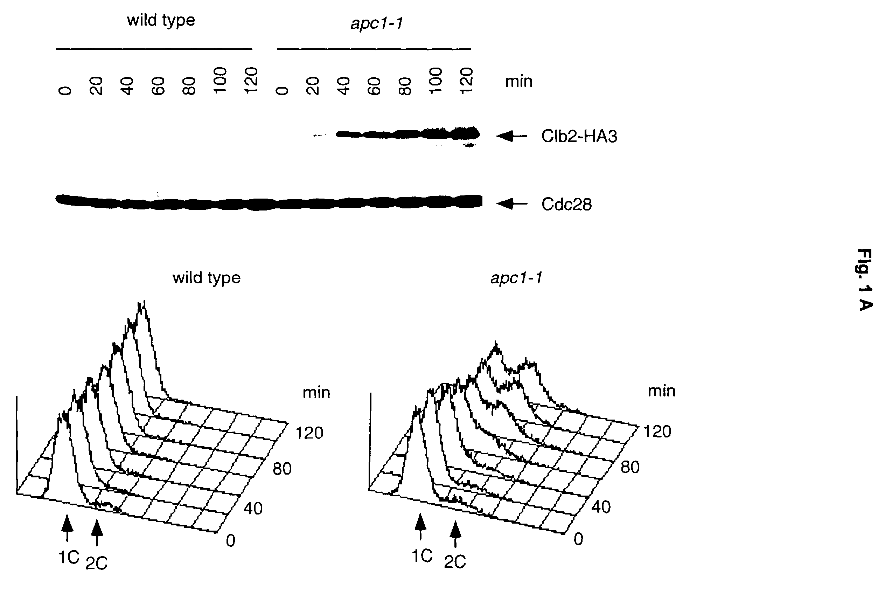

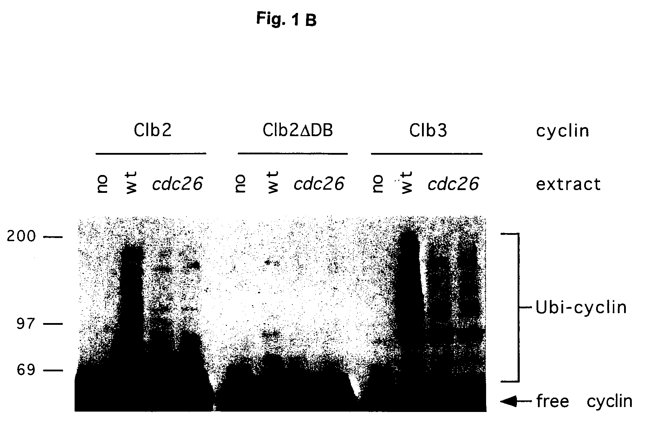

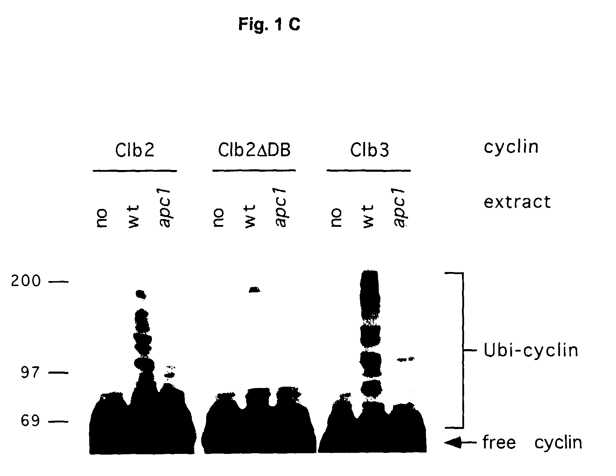

The screening methods of the invention are based on the principle to determine the ability of the APC to ubiquitinate a cyclin B containing substrate. This principle is described in the experiment shown in Fig. 1 of Zachariae and Nasmyth, 1996, in which yeast extracts that are capable of cell cycle regulated and destruction box-dependent ubiquitination were employed to show the ubiquitination of Clb2 in extracts from yeast strains overexpressing Clb2.

To perform such a screen, the recombinant APC is incubated in a reaction containing a ubiquitin activating enzyme (E1), a suitable ubiquitin conjugating enzyme (E2), ubiquitin, ATP and a substrate (e. g. cyclin B) which can be detected either by immunoblotting or because it is labeled radioactively. Cyclins containing a mutated or deleted destruction box can be used as negative control.

To convert this type of ubiquitination assay into a high throughput screening system, microtiter plates are coated with an APC substrate such as a cyclin B. The cyclins may be produced in recombinant form (see e.g. Glotzer et al., 1991); alternatively cell free extracts from cells overexpressing the respective cyclins may be used instead of the pure cyclins. The substrate is incubated with APC in the presence of ubiquitin-activating enzyme and ubiquitin conjugating enzyme (Rolfe et al., 1995), ATP, an epitope-tagged ubiquitin (Ellison and Hochstrasser, 1991) and the test substance, and the amount of immobilized ubiquitin is measured using an enzyme (e. g. alkaline phosphatase or beta-galactosidase) linked to the anti-tag antibody which produces a color reaction. Substances that reduce the amount of immobilized ubiquitin are candidates for drugs that interfere with the cell cycle and thus inhibit the proliferation of rapidly proliferating cells, in particular tumor cells.

The yeast APC can be used in an initial screen for substances which interfere with the cell cycle in yeast. The ability of such substances to interfere with the ubiquitination of type B cyclins in higher eucaryotic cells, in particular in human cells, and therefore to inhibit rapidly proliferating cells, may then be confirmed in a secondary assay that employs the higher eucaryotic, in particular human, APC.

The present invention is based on the following findings and considerations:

Mitotic cyclins such as Clb2p are rapidly degraded in G1 arrested yeast cells (Irniger et al., 1995; Amon et al., 1994). To isolate mutants defective in cyclin proteolysis, in the experiments of the present invention mutagenized colonies were screened for β-galactosidase activity due to the accumulation of a Clb2-lacZ protein in G1 at 37°C as described (Irniger et al., 1995). 18 mutants were identified that arrested as large budded cells with a 2C DNA content after cycling cultures were shifted from 25 to 37°C. Complementation analysis and gene cloning demonstrated that new alleles of *CSE1* (Irniger et al., 1995), *CDC16* (King et al., 1995), *CDC26* (*cdc*26-*519*) and a mutant allele of a new gene have been isolated. The new gene was called *APC1*. *cse1* and *cdc16* mutants were previously identified in a similar screen (Irniger et al., 1995). The *CDC26* gene encodes an acidic protein of 17 kD (Akari et al., 1992).

Induction of a *GAL* promoter-*CLB2*-*HA3* fusion in G1 arrested cells at 35°C led to the accumulation of Clb2-HA3p within 20 min in the *apc1-1* mutant but not in wild-type cells. Mutant cells initiated S phase after 60 min but failed to bud whereas wild-type cells stayed arrested (Fig. 1A). A similar phenotype is caused by expression of a non-destructible version of Clb2p in wild-type cells (Amon et al., 1994). Extracts prepared from G1-arrested *cdc26-519* and apc*1-1* mutants were defective in ubiquitination of the mitotic cyclins Clb2p and Clb3p (Fig. 1, B and C), suggesting that reduced Clb2p proteolysis in the mutants stems from defective ubiquitination.

At 37°C *cdc26-519* mutants arrested as ]arge-budded cells containing a short mitotic spindle in an undivided nucleus positioned at the bud-neck. Haploid cells lacking the entire *CDC26* coding sequence were viable at 25°C but arrested with a similar phenotye at 37°C. *CDC16-HA3 CDC26-myc9* cells grown at 25°C were shifted to 37°C and samples were taken for immunoblot analysis. Within two hours after the temperature shift the amount of Cdc26-myc9p increased 10-fold. The amount of Cdc16-HA3p did not increase.

The *APC1* wild-type gene encodes a 1748-amino acid (192-kD) protein whose COOH-terminal half is similar to the BIME protein from *A. nidulans* (Engle et al., 1990) and to a related protein encoded by the mouse *tsg24* gene (Starborg et al., 1994). An alignment of amino acids 904 to 1713 of Apc1p shows 28% and 25% identity to the corresponding regions of BIMEp (aa 1217 to 1939) and Tsg24p (aa 1026 to 1777), respectively. Tetrad analysis of spores derived from a diploid in which one copy of *APC1* was replaced by *HIS3* showed that *APC1* is an essential gene. His⁺ spores arrested as large budded cells after one or two cell divisions after germination.

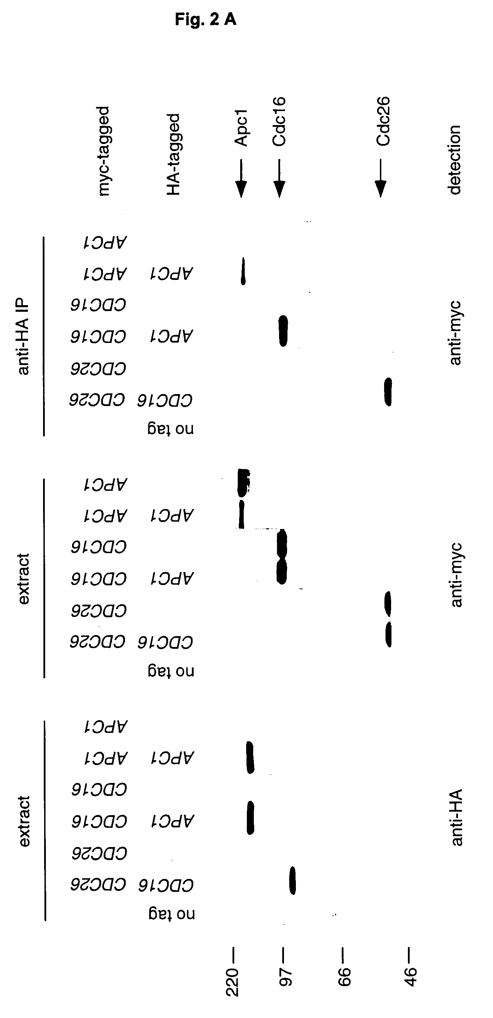

Cdc16p, Cdc23p, and Cdc27p associate with each other in yeast (Lamb et al., 1994). Cyclin proteolysis in vivo and cyclin ubiquitination in vitro also depends on the Cse1 protein (Irniger et al., 1995; Zachariae and Nasmyth, 1996). To investigate whether Cdc26p, Apc1p, and Cse1p associate with the Cdc16p-Cdc23p-Cdc27p complex, the endogenous genes were modified to encode variants carrying COOH-terminal HA (hemagglutinin) or myc epitope tags. All of these variants were fully functional. Extracts were prepared from strains expressing two proteins with different epitope tags and subjected to immunoprecipitations with the antibody to HA (Fig. 2A). Cdc16-myc6p was co-precipitated with Apc1 -HA3p but not with Cse1-HA3p. Cdc26-myc9p was co-precipitated with Cdc16-HA3p. Apc1-myc6p was co-precipitated with Apc1-HA3p from an extract prepared from an *APC1-HA3/APC1-myc6* diploid strain. To characterize the complex further, various strains, each expressing a different myc-tagged protein, were labeled with ³⁵S methionine and cysteine. The antibody to the myc epitope precipitated the same set of proteins from extracts of *CDC16-myc6*, *CDC23-myc9*, *CDC26-myc9*, *CDC27-myc9* and *APC1*-*myc18* cells. This set included Cdc16p, Cdc23p, Cdc27p and Apc1p, which were identified by the increased molecular weight of epitope-tagged variants, and a protein of 80 kD. A protein with a size close to that of Cdc16p (100 kD) was detected in the immunoprecipitate from the *CDC16*-*myc6* strain. None of these proteins were co-precipitated with Cse1-myc9p. These data together with previous work (Zachariae and Nasmyth, 1996; Lamb et al., 1994) suggest that cyclin ubiquitination in yeast depends on a complex containing at least two molecule each of Apc1p, Cdc16p, Cdc23p, and Cdc27p. Cdc26p and at least two unidentified proteins (p80 and p100) are also components of this complex.

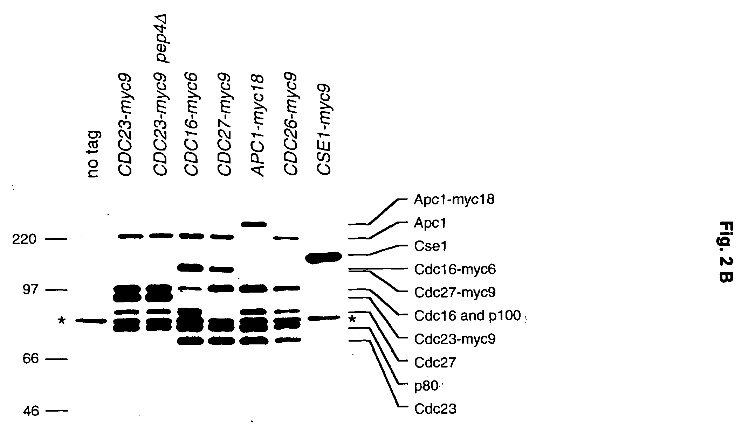

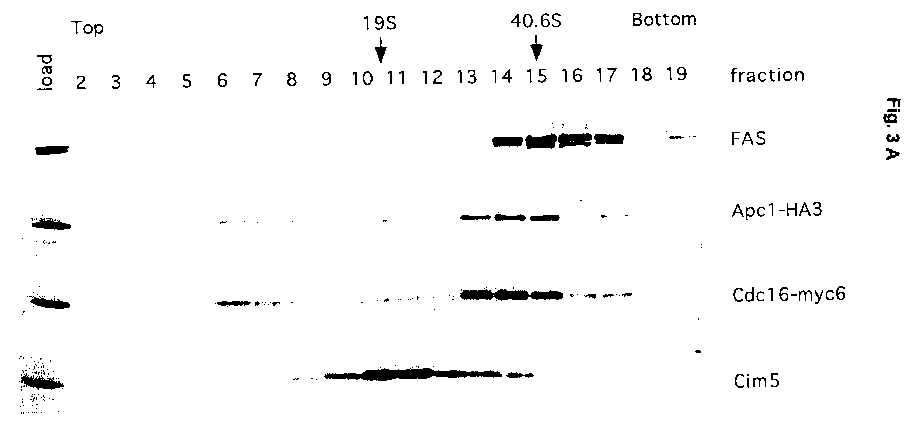

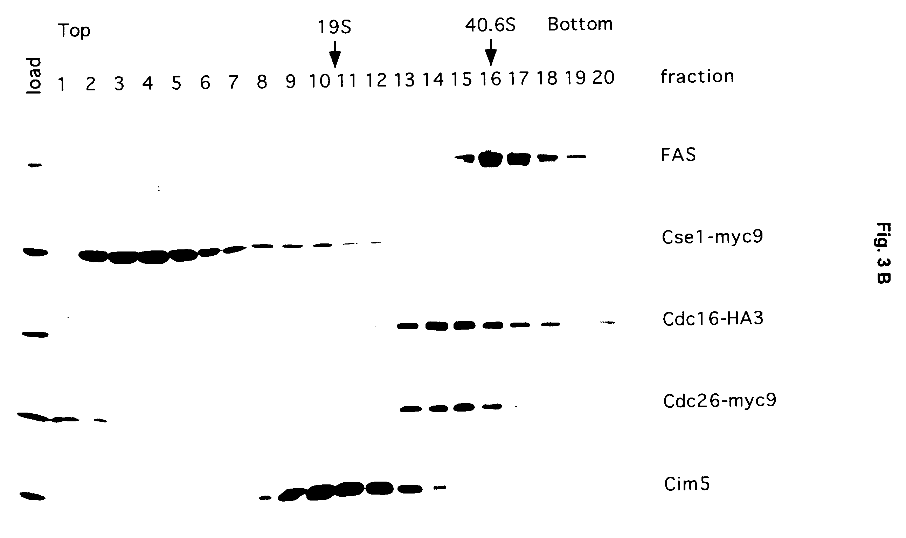

To determine the size of the yeast APC, extracts from strains expressing two epitope-tagged proteins were analyzed by glycerol density gradient centrifugation. Apc1-HA3p co-sedimented with Cdc16-myc6p as a 36S particle [Fig. 3A]. Cdc23-HA3p co-sedimented with Cdc16-myc6p with the same velocity. The size of the yeast complex is larger than that reported for the *Xenopus* APC (20S) (King et al., 1995) and the cyclosome from clam (Sudakin et al., 1995). Cdc26-myc9p but not Cse1-myc9p co-sedimented with Cdc16-HA3p (Fig. 3B). These results and the immunoprecipitation data indicate that Cse1p is not a component of the APC.

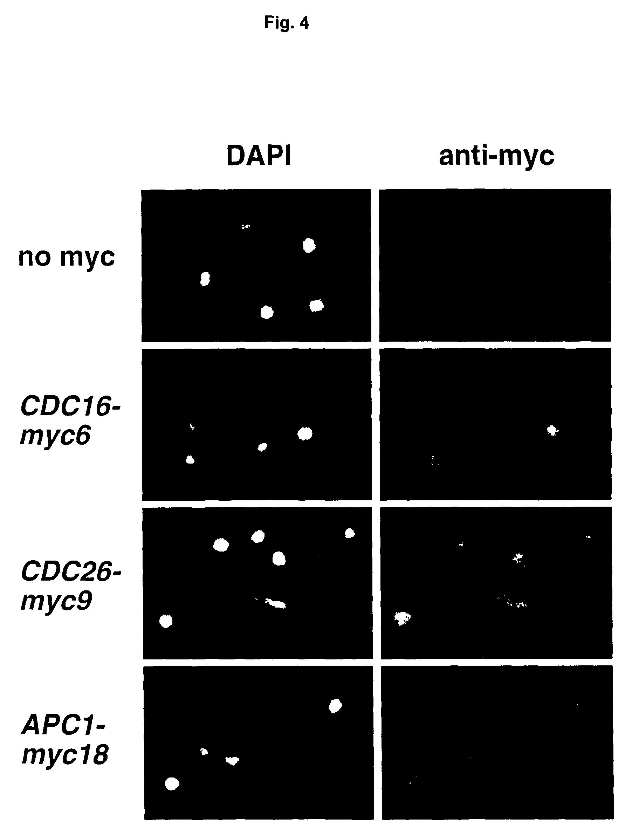

The sub-cellular localisation of the APC components was determined by indirect immunofluorescence. Cdc16-myc6p, Cdc26-myc9p, and Apc1-myc18p are localized in the nucleus (Fig.4). Cdc23-myc9p, Cdc27-myc9p and Cse1-myc9p were also found to be nuclear proteins. Specific accumulation of any of these proteins at spindle pole bodies or on mitotic spindles was not detected.

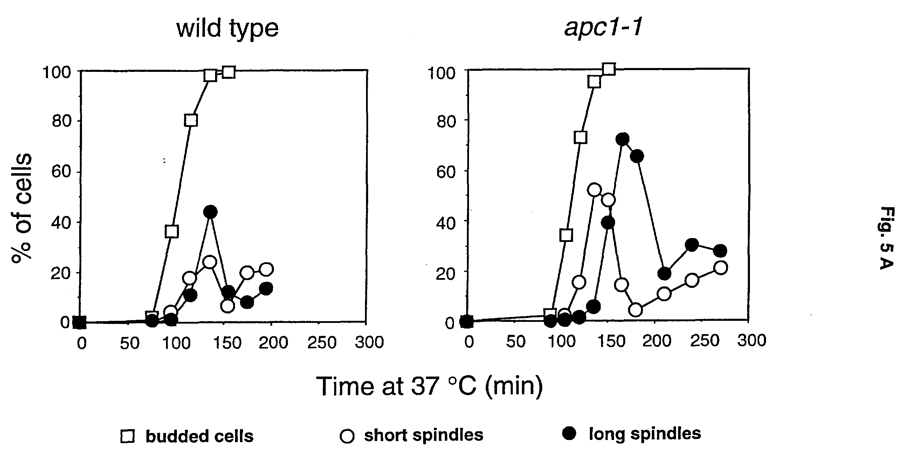

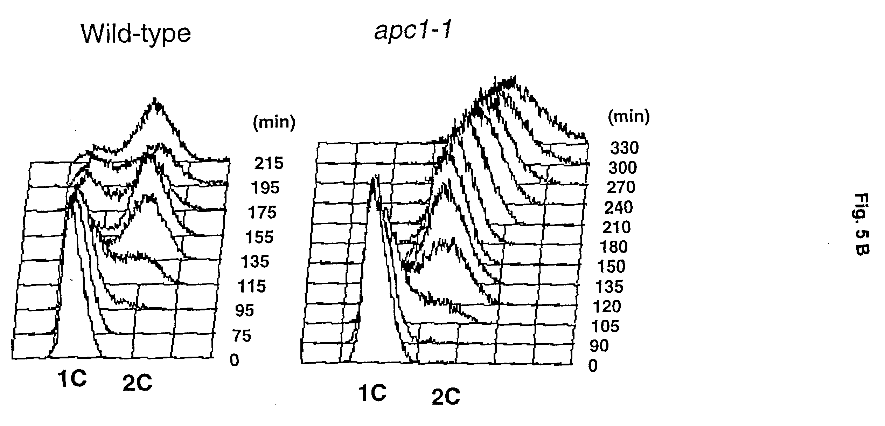

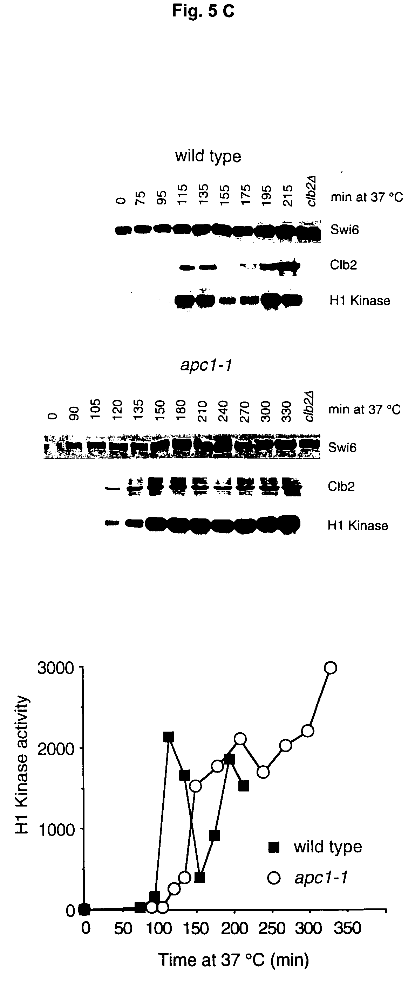

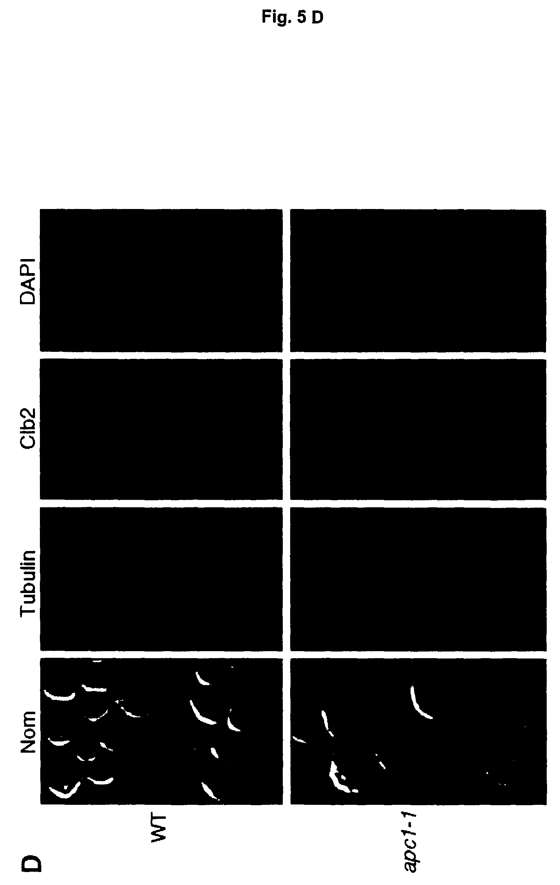

To investigate the role of *APC1* in vivo, small, unbudded G1 cells were isolated from wild-type and *apc1-1* cultures grown at 25°C, and their progression was followed through the cell cycle upon incubation at 37°C. In *apc1-1* cells DNA replication, budding, and the formation of mitotic spindles occurred at the same time as in wild-type cells but entry into anaphase was delayed by approximately 20 min (Fig. 5, A and B). Most *apc1-1* cells later underwent anaphase but were slow in disassembling their mitotic spindles. They eventually rebudded without undergoing cytokinesis or re-replication. It conclude that *apc1-1* cells are defective in the onset of anaphase, in the final exit from mitosis, and in the completion of cytokinesis. In the *apc1-1* mutant, Clb2 protein and Clb2p-associated histone H1 kinase activity appeared later than in wild-type cells. This finding could explain why entry into anaphase is delayed in the mutant. Neither Clb2 protein nor Clb2p-Cdc28p kinase activity declined as mutant cells underwent anaphase (Fig. 5C). The observation that *apc1-1* cells rebud without any apparent drop in kinase activity is surprising because high Clbp-Cdc28p kinase activity is thought to inhibit rebudding (Dahman et al., 1995).

To detect Clb2p in individual cells by indirect immunofluorescence microscopy, the endogenous *CLB2* genes of a wild-type and an *apc1-1* strain were replaced by the epitope-tagged variant *CLB2*-*myc12*. G1 cells, obtained by growth to stationary phase at 25°C, were inoculated into fresh medium at 37°C causing cells to re-enter the cell cycle. In wild-type cells Clb2-myc12p accumulated in maximal amounts at the onset of anaphase and then rapidly declined as cells underwent nuclear division. In the *apc1-1* mutant the amount of Clb2-myc12p remained high in cells containing separated chromosomes and fully elongated spindles (Fig. 5D). It was concluded that Apc1p is required for cyclin proteolysis not only in G1 but also in late anaphase-telophase. Surprisingly, apc*1-1* cells were defective in the formation of astral microtubules emanating from the poles of mitotic spindles (Fig. 5D). in contrast, *cdc16-123* cells arrested at 37°C had normal astral microtubules.

The accumulation of Cdc26p at high temperature is consistent with the observation that Cdc26p function is only essential for APC activity at 37°C. Cdc26p may be required to stabilize the APC or to modulate its activity under conditions of stress, such as heat shock. The discovery that the BIMEp homologs of yeast (Apc1p) is a subunits of the APC explains the pre-anaphase arrest of *bimE* mutants. Loss of *bimE* function partially bypasses the control mechanisms that render entry into mitosis dependent on the completion of DNA replication and on the activation of the NIMA kinase (Osmani et al., 1995). Taken together these data indicate that the APC is not only required for the onset of anaphase and the exit from mitosis but may also be involved in regulating entry into mitosis.

### Brief description of the figures:

- Fig. 1A:: Accumulation of Clb2p in G1-arrested *apc1-1* cells. Immunoblot and DNA content at different time points
- Fig. 1B:: Extracts from G1-arrested *cdc26-519* cells are defective in the ubiquitination of mitotic cyclins. Immunoblot of reactions with extracts from G1-arrested wild type and *cdc26-519* cells.
- Fig. 1C:: C: Extracts from G1-arrested *apc1-1* cells are defective in the ubiquitination of mitotic cyclins. Immunoblot.
- Fig. 2A:: Co-immmunoprecipitation of Apc1p and Cdc26p with other subunits of the APC.
- Fig. 2B:: Precipitation of the APC from metabolically labeled cells
- Fig. 3A:: Determination of the size of the yeast APC. Apc1p and Cdc16p co-sediment as a particle of 36S.
- Fig. 3B:: Determination of the size of the yeast APC. Cdc26p co-sediments with Cdc16p as a 36S particle. Cse1p does not co-sediment with the APC.
- Fig. 4:: Nuclear localisation of subunits of the yeast APC. Detection of Cdc16-myc6p, Cdc26-myc9p, and Apc1-myc18p by indirect immunofluorescence.
- Fig. 5A:: Role of *APC1 in vivo*. Defective anaphase in the *apc1-1* mutant.
- Fig. 5B:: Role of *APC1 in vivo*. Distribution of the DNA content. Comparison of DNA replication in *apc1-1* cells and in wild type cells.
- Fig. 5C:: Role of *APC1 in vivo*. Clb2 protein and Clb2p-associated kinase activity.
- Fig. 5D:: Role of *APC1 in vivo*. Defective Clb2p destruction and lack of astral microtubules in *apc1-1* cells.
- Fig. 6A:: Apc1p, BIMEp and Tsg24p belong to a family of homologous proteins. Dot blot comparison.
- Fig. 6B:: Sequence alignment of the C-terminal homologous regions of Apc1p, BIMEp and Tsg24p

In the following examples, unless stated otherwise, standard methods for DNA manipulation were used as described by Ausubel et al., 1994. Unless stated otherwise, manipulation of yeast was carried out as described by Guthrie and Fink, 1991.

### Example 1

### Isolation of mutants defective in cyclin degradation and cloning of the respective genes

Mutants defective in the proteolysis of the mitotic cyclin Clb2p were isolated as described recently (Irninger, et al., 1995). Briefly, a Δ*cln1,2,3 MET3* promoter-*CLN2 GAL1-10* promoter-*CLB2-lacZ* strain (K3828) was mutagenised with EMS. Cells were first arrested in G1 and then induced to express a Clb2-lacZ protein at 37°C by sequentially transferring colonies to medium containing methionine and to medium containing methionine plus galactose, respectively. Colonies (400,000) were screened for beta-galactosidase activity due to the accumulation of the Clb2-lacZ protein. Potential mutants were grown at 25°C and subsequently shifted to 37°C. The cellular DNA content was analysed by flow cytometry and mutants that arrested as large budded cells with a 2C DNA content were further analysed. The original mutants were back-crossed at least three times to wild type.

Complementation analysis and gene cloning demonstrated that new alleles of *CSE1* (7 alleles), *CDC16(*4 alleles), *CDC26* (*cdc26-519*) and a mutant allele of a new gene had been isolated. The new gene was called *APC1*. For gene cloning, mutants were transformed with a genomic library (Cvrckova and Nasmyth, 1993) and plasmids allowing growth at 37°C were recovered. The temperature sensitive lethality of mutant 519 was complemented by the *CDC26* gene (Akari et al., 1992) present on a plasmid containing sequences from chromosome VI. A *LEU2* marker was integrated at the genomic *CDC26* locus and the resulting *CDC26::LEU2* strain was crossed to a *cdc26-519* mutant All 17 tetrads derived from this cross were parental ditypes demonstrating that the cloned gene is closely linked to the *cdc26-519* mutation.

The *apc1-1* mutation was complemented by the open reading flame YNL172W (*Saccharomyces* Genome Database) present on two plasmids containing different but overlapping sequences from chromosome XIV. A *HIS3* marker was integrated at the genomic *APC1* locus and the resulting *APC1*-*HA3*-*HIS3* strain was crossed to an *apc1-1* mutant. All 38 tetrads were parental ditypes indicating that the cloned gene is closely linked to the *apc1-1* mutation.

The *APC1* wild-type gene encodes a 1748-amino acid (192-kD) protein whose COOH-terminal half is similar to the BIME protein from *A. nidulans* (Engle et al., 1990) and to a related protein encoded by the mouse *tsg24* gene (Starborg et al., 1994).

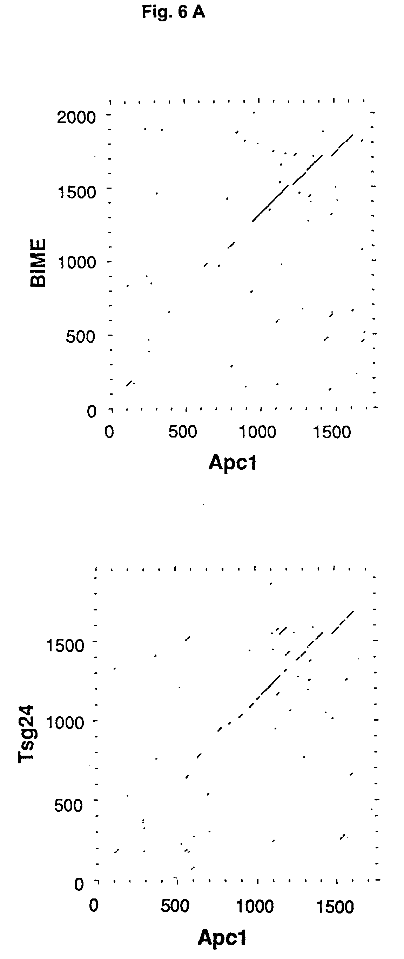

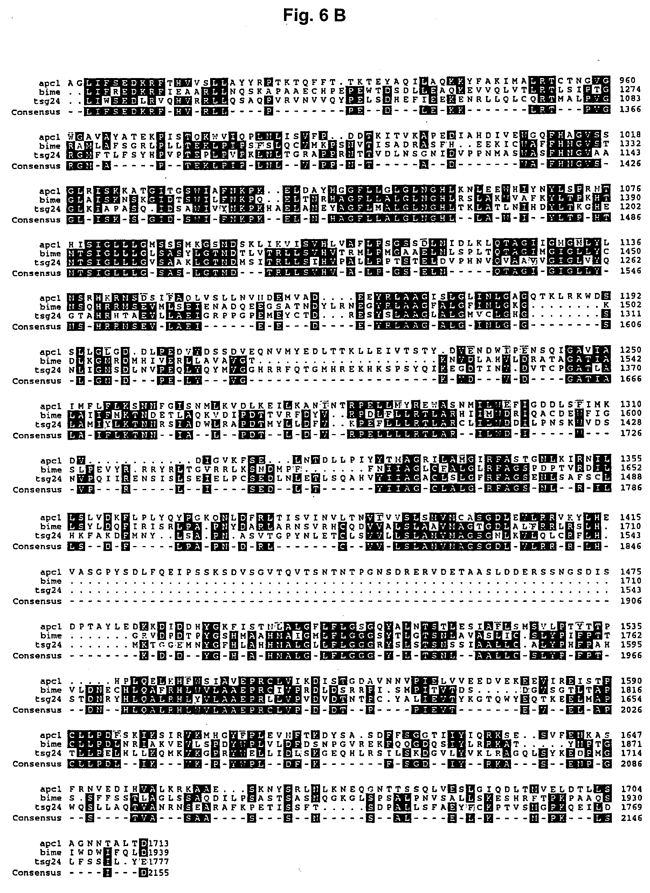

Fig. 6 shows that Apc1p, BIMEp and Tsg24p belong to a family of homologous proteins. Fig. 6A shows a dot blot comparison of Apc1p with BIMEp (top) and Apc1p with Tsg24p (bottom). The COMPARE program (Window 50, Stringency 23) of the GCG software package was used for sequence coparison.

Fig. 6B shows the sequence alignment of the C-terminal homologous regions of Apc1p, BIMEp and Tsg24p. Identical amino acids are indicated with black boxes. The PILEUP program of the GCG sofware was usen.

### Example 2

### Defective proteolysis and ubiquitination of mitotic cyclins in the cdc26-519 and apc1-1 mutants

### A: Accumulation of Clb2p in G1-arrested apc1-1 cells

Wild-type (K5137) and *apc1-1* cells (K6221) of the genotype *MATa GALp-CLB2-HA3 bar*1 were grown at 23°C in 400 ml YEP medium (1% yeast extract, 1% peptone) containing 2% raffinose to a cell density of 0.3 x 10⁷ cells/ml. Cells were arrested in G1 by addition of alpha-factor (0.5 µg/ml) to the medium for 5 h. At time point 0, cells were transferred to YEP medium containing 2% raffinose plus 2% galactose and alpha-factor (0.5 µg/ml) at 35°C. Samples for immuno-blotting (40 ml culture) and flow cytometric analysis of cellular DNA content (2 ml culture) were withdrawn at the indicated time points. Protein extracts were prepared and Clb2-HA3p was detected by immuno-blotting using the monoclonal antibody 12CA5 directed against the HA epitope tag. Cdc28p was detected as a loading control using a rabbit antiserum. Preparation of protein extracts and immunoblotting were carried out as described (Surana, et al., 1993). Bands were detected with the enhanced chemiluminescence system (ECL, Amersham). A Becton-Dickinson FACScan was used for analysis of cellular DNA content (Epstein and Cross, 1992). Fig. 1A shows the immunoblot and the DNA content at different time points. Clb2-HA3p accumulated in the *apc1-1* cells but not in wild type cells. *apc1-1* cells initiated DNA replication whereas wild type cells stayed arrested.

### B: Extracts from G1-arrested cdc26-519 cells are defective in the ubiquitination of mitotic cyclins

A wild type (K5518) and two *cdc26-519* strains (K6200 and YWZ135) (all strains are *MATa pep4 bar1*) were grown at 25°C in 300 ml YEP medium containing 2% raffinose and 50 mM sodium phosphate pH 6.5 to a cell density of 0.55 x 10⁷ cells/ml. Cells were arrested in G1 with alpha-factor (0.5 µg/ml) for 2.5 h at 25°C and then shifted to 37°C for 1.5 h.

Protein extracts for the ubiquination assay and the ubiquitination reaction were prepared essentially as described (Zachariae and Nasmyth, 1996). Cells were collected on membrane filters and incubated in 30 ml of Tris-SO₄/DTT solution or 3 min. Cells were washed with 30 ml spheroplast medium containing 0.5% raffinose and 0.5 µg/ml alpha-factor (pre-warmed to 37°C) and incubated in the same medium containing 0.5 mg lyticase (Sigma, L5763) for 10 min at 37°C. The following steps were carried out at 0 to 4 °C. The spheroplast suspension was diluted to 50 ml with ice cold 1.2 M sorbitol. Spheroplasts were collected by centrifugation, resuspended in 50 ml sorbitol and collected again. The spheroplasts were resuspended in 0.2 ml of buffer A and disrupted by shaking with 0.3 ml of glass beads (0.5 mm) for 4 min. The lysate was cleared by centrifugation for 5 min in an Eppendorf micro-centrifuge and the supernatant was used directly for the ubiquitination assay. Protein concentrations were determined with the Bio-Rad protein assay using bovine serum albumin as a standard. The protein concentrations of the extracts used for this experiment were 50 mg/ml.

Extracts containing the cyclin-substrates were prepared as described above from strains overexpressing HA3-tagged Clb2p, Clb2p with a deletion of the destruction box (Clb2ΔDBp) (Zachariae and Nasmyth, 1996), and Clb3p. Extracts were frozen in aliquots (20 µl) in liquid nitrogen and stored at -80°C. The protein concentration of these extracts was 40 mg/ml.

32 µl extract was mixed with 4 µl ATP-regenerating system in a total volume of 38 µl. 2 µl of extract containing the cyclin substrates Clb2-HA3p, Clb2ΔDB-HA3p or Clb3-HA3p was added and the reaction was incubated for 5 min at room temperature. Reactions were terminated by diluting samples 1:5 into hot SDS sample buffer. 25 µl samples were separated by electrophoresis on 8% SDS-polyacrylamide gels and subsequently blotted to a membrane. HA-tagged cyclin and cyclin-ubiquitin conjugates were detected with the monoclonal antibody 12CA5 and an enhanced chemoluminescence detection system purchased from Amersham.

Fig. 1B shows the immunoblot of reactions with extracts from G1-arrested wild type and *cdc26*-*519* cells. The wild type extract conjugates ubiquitin (which is present in the extract in sufficient amouts) to Clb2-HA3p and Clb3-HA3p but not to Clb2ΔDB-HA3p. The extracts from the mutant strains are defective in ubiquitination of Clb2-HA3p and Clb3-HA3p.

### C: Extracts from G1-arrested apc1-1 cells are defective in the ubiquitination of mitotic cyclins

A wild type (K1771) and an *apc1-1* strain (K6199) (both strains have the genotype *MATa pep4 bar1*) were grown at 23°C in 300 ml YEP medium containing 2% raffinose and 50 mM sodium phosphate pH 6.5 to a cell density of 0.55 x 10⁷ cells/ml. Cells were arrested in G1 with alpha-factor (0.5 µg/ml) for 4.5 h at 23°C and then shifted to 37°C for 40 min. The G1-arrest was confermed by flow cytometric analysis of the cellular DNA content. Protein extracts were prepared and used for ubiquitination reactions as described above. The protein concentrations of the extracts used in this experiment were 30 mg/ml. Fig. 1C shows the immunoblot of the reactions. The extract from the *apc1-1* mutant is defective in the ubiquitination of the cyclin substrates Clb2-HA3p and Clb3-HA3p. Example 3

### Disruption of the APC1 and CDC26 genes

### A: APC1 is an essential gene

A plasmid was constructed in which a 4.3-kb Bam HI to Sty I fragment within the APC1 gene was replaced by *HIS3*. The *apc1::HIS3* construct was transformed into a diploid wild type strain. Clones in which one *APC1* allele was disrupted by *HIS3* were identified by Southern (DNA) blot analysis of genomic DNA isolated from His⁺ transformants. An *apc1::HIS3/APC1* strain was sporulated and tetrads were dissected. His⁺ spores arrested as large budded cells after one or two cell divisions after germination whereas His⁻ spores grew normally. These data show that *APC1* is an essential gene.

### B: CDC26 is only essential at high temperature

A method using fragments generated by polymerase chain reaction (PCR) to disrupt genes with the *URA3* gene from *Kluyveromyces lactis* is described by Längle-Rouault and Jacobs, 1995.

A fragment was amplified by polymerase chain reaction (PCR) containing the *Kluyveromyces lactis URA3* gene flanked by two *CDC26* sequences of 45 bp which are located upstream and downstream of the start and stop codon, respectively. The PCR fragment was transformed into a diploid wild type strain. Clones in which one *CDC26* allele was replaced by the *K. lactis URA3* gene were identified by PCR-amplification of the *CDC26* locus form genomic DNA isolated from Ura⁺ transformants. A *Δcdc26::KlURA3/CDC26* strain was sporulated and tetrads were dissected. Tetrads consisted of two Ura⁺ spores which were viable at 25°C but arrested as large budded cells at 37°C and two Ura⁻ spores which grew normally at 37°C. These data show that *CDC26* is only essential for poliferation at 37°C.

### Example 4

### Analysis of the subunit composition of the APC

### A: Epitope tagging

To investigate whether Cdc26p, Apc1p, and Cse1p associate with the Cdc16p-Cdc23p-Cdc27p complex, the endogenous genes were modified to encode variants carrying COOH-terminal HA or myc epitope tags: Two fragment emcopassing 200 bp of coding region upstream of the stop codon and 200 bp of 3'-non-coding region downstream of the stop codon, respectively , were amplified by PCR. The PCR-primers were choosen to introduce an artificial restriction site in front of the stop codon. The two fragments were ligated into a yeast integrative vector and fragments encoding several HA (three HA epitopes) or myc epitope tags (six or nine myc epitopes) were inserted into the restriction site in front of the stop codon. The following yeast integrative vectors were used: YIplac128 (*LEU2*), YIplac204 (*TRP1*), YIplac211 (*URA3*) (Gietz and Sugino, 1988); pRS303 (*HIS3*), (Sikorski and Hieter, 1989). The resulting plasmid was transformed into yeast. Homologous recombination into the respective genomic locus generates an epitope-tagged full-length version of the gene linked to a selectable marker. Epitope-tagged *CDC16*, *CDC23* and *CDC27* were marked with *URA3*, *LEU2* and *TRP1*, respectively. Tagged *CDC26, APC1* and *CSE1* were each marked with *HIS3*. Homologous integration of the plasmid was verified by Southern (DNA) blot analysis and detection of the epitope tagged protein by immunoblotting using anti-HA or anti-myc antibodies. Strains expressing two epitope-tagged proteins were obtained by genetic crosses. At 37°C all strains grew normally demonstrating that the epitope-tagged proteins were fully functional.

### B: Co-immmunoprecipitation of Apc1p and Cdc26p with other subunits of the APC

Extracts were prepared from strains expressing a HA-tagged protein and a myc-tagged protein (e.g. a n *APC1-HA3 CDC16-myc6* strain). Extracts were subjected to immunoprecipitations with the antibody to HA and co-precipitation of the myc-tagged protein was analysed by immunoblotting. Extracts prepared from strains expressing only the respective myc-tagged protein (e.g. a *CDC16*-*myc6* strain) were used as controls.

Extracts for immunoprecipitations were prepared as described (Lamb, et al., 1994). Strains were grown at 30°C in YEP medium (1% yeast extract, 1% peptone) containing 2% glucose to a cell density of 0.7 x 10⁷ cells/ml. 2 x 10⁹ cells were lysed with glass beads in 0.4 ml buffer A (50 mM Tris-HCl pH 7.5, 50 mM NaCl, 0.2% Triton X100, 10% glycerol, 1 mM DTT, 1 mM PMSF, 1 µg/ml of leupeptin and pepstatin). Extracts were cleared by centrifugation (20 min 12,000 x g). 0.3 ml extract adjusted to a protein concentration of 20 mg/ml was incubated for 1 h at 4°C with 30 µl of a 50% suspension of 12CA5 (anti-HA) antibody crosslinked to protein A-Sepharose. Crosslinking was carried out as described by Harlow and Lane, 1988. The beads were washed three times with 1 ml of buffer A and bound proteins were released by addition of SDS to 3%. After removal of the beads by filtration, samples were analyzed by immuno-blotting using anti-HA (12CA5) or anti-myc (9E10) monoclonal antibodies.

Immunoblots of co-precipitation experiments are shown in Fig. 2A. Cdc16-myc6p was co-precipitated with Apc1-HA3p. Cdc26-myc9p was co-precipitated with Cdc16-HA3p. Apc1-myc6p was co-precipitated with Apc1-HA3p from an extract prepared from an *APC1-HA3/APC1-myc6* diploid strain. These data together with previous work (Zachariae and Nasmyth, 1996; Lamb, et al., 1994) suggest that cyclin ubiquitination in yeast depends on a complex containing at least two molecules each of Apc1p, Cdc16p, Cdc23p, and Cdc27p. Cdc26p is also components of this complex.

### C: Precipitation of the APC from metabolically labeled cells

To characterize the complex further, various strains, each expressing a different myc-tagged protein, were labeled with ³⁵S methionine and cysteine. Extracts were prepared and subjected to immunoprecipitations using the antibody to the myc epitope tag. Precipitated proteins were separated by SDS-polyacrylamide gelelectrophoresis and detected by autoradiography.

Strains were grown at 30°C in synthetic complete medium lacking methionine. 5 x 10⁷ cells were labeled with 1 mCi ³⁵S methionine/cysteine in 1.5 ml medium for 2 hours at 30°C (Sherman, 1991). Cells were broken with glass-beads in 0.25 ml buffer B [buffer A (Zachariae and Nasmyth, 1996) diluted 1:3] containing 1.5 mg unlabeled protein from a *pep4* strain (K5517) (Zachariae and Nasmyth, 1996). After centrifugation (10 min, 12,000 g), extracts were sequentially incubated with protein A-sepharose (160 µl) and 9E10 antibody crosslinked to protein A-sepharose (25 µl). The beads were washed with buffer B (4 x 1 ml), buffer B with 120 mM K-acetate (1 ml) and buffer B with 150 mM K-acetate (1 ml). Bound proteins were released by addition of SDS to 3%, heated in SDS-sample buffer and separated on 8% SDS-polyacrylamide gels. Gels were treated with Entensify solution (Du Pont de Nemours), dried on filter paper and exposed to X-OMAT film (Kodak) for 12 h.

An autoradiograph is shown in Fig. 2B. The same set of proteins was precipitated from extracts of *CDC16-myc6*, *CDC23-myc9*, *CDC26-myc9*, *CDC27-myc9* and *APC1-myc18* cells. This set included Cdc16p, Cdc23p, Cdc27p and Apc1p, which were identified by the increased molecular weight of epitope-tagged variants, and a protein of 80 kD. A protein with a size close to that of Cdc16p (100 kD) was detected in the immunoprecipitate from the *CDC16-myc6* strain. None of these proteins were co-precipitated with Cse1-myc9p.

These data suggest that the yeast APC contains, in addition to Apc1p, Cdc16p, Cdc23p, Cdc27p, and Cdc26p, at least two unidentified proteins (p80 and p100). The Cse1 protein is not a component of the APC.

### Example 5

### Co-sedimentation of Apc1p, Cdc16p, and Cdc26p as a 36S particle

To determine the size of the yeast APC, extracts from strains expressing two epitope-tagged proteins were analysed by glycerol density gradient centrifugation.
A: An *APC1-HA3 CDC16-myc6* strain (K6190) was grown at 30°C in 200 ml of YEP medium containing 2% raffinose to a cell density of 0.7 x 10⁷ cells/ml. Cells were broken with glass beads in 0.4 ml buffer B (50 mM Hepes-KOH pH 7.3, 60 mM K-acetate, 5 mM Mg-acetate, 0.1% Triton X100, 1 mM DTT, 1 mM PMSF, 1µg/ml of leupeptin and pepstatin) containing 5% glycerol. The lysate was cleared by centrifugation (20 min 12,000 x g). 0.15 ml extract (2.4 mg protein) was layered on a 10-35% glycerol gradient in buffer B and centrifuged for 15 h at 28,000 rpm in a Beckman SW40 rotor at 4°C. 20 fractions were collected from the top and proteins were precipitated with TCA. Proteins were dissolved in SDS sample buffer, separated in 7% SDS-polyacrylamide gels and analysed by immunoblotting. Apc1-HA3p and Cdc16-myc6p were detected with the antibodies 12CA5 and 9E10, respectively, and an enhanced chemoluminescence detection system purchased from Amersham. Cim5p, a subunit of the 19S proteasome activator complex, and fatty acid synthetase (FAS, 40.6S) were detected with polyclonal rabbit antisera. The immunoblot is shown in Fig. 3A. Apc1p and Cdc16p co-sediment as a particle of 36S.
B: Extracts from *CDC16-HA3, CSE1-myc9* (K6184) and *CDC16-HA3 CDC26-myc9* (K6323) strains were prepared and separated on parallel gradients as described in (A). The immunoblot is shown in Fig. 3B. Cdc26p co-sediments with Cdc16p as a 36S particle. Cse1p does not co-sediment with the APC.

### Example 6

### Nuclear localisation of subunits of the yeast APC

Control cells (no myc, K1534) and cells containing *CDC16-myc6* (K6180), *CDC26-myc9* (K6322), or *APC1-myc18* (K6329) were grown in YEP medium containing 2% glucose at 30°C. Cells were fixed with formaldehyde and prepared for indirect immunofluorescence. Myc-tagged proteins were detected with the 9E10 antibody and a CY3-conjugated secondary antibody. DNA was stained with DAPI. Cells were examined with a Zeiss Axiophot microscope and pictures were taken on Kodak T-MAX400 film. Cdc16-myc6p, Cdc23-myc9p, Cdc26-myc9p, Cdc27-myc9p and Apc1-myc18p are all localized in the nucleus. Cse1-myc9p was also found to be a nuclear protein. Fig. 4 shows the detection of Cdc16-myc6p, Cdc26-myc9p, and Apc1-myc18p by indirect immunofluorescence.

### Example 7

### Defective anaphase in the apc1-1 mutant

To investigate the role of *APC1 in vivo*, we isolated small, unbudded G1cells from wild type and *apc1-1* cultures grown at 25°C and followed their progression through the cell cycle upon incubation at 37°C.
A wild-type (K699) and an *apc1-1* strain (K5717) were grown at 25°C in synthetic complete medium containing 2% raffinose to increase the fraction of G1 cells. Small unbudded G1 cells were isolated by centrifugal elutriation as described (Schwob and Nasmyth, 1993). Small G1 cells were released into YEP medium containing 2% glucose at 37°C and samples were withdrawn at the indicated time points. The fraction of budded cells was determined by microscopic examination of sonicated samples. Mitotic spindles were detected by indirect immunofluorescence. A Becton-Dickinson FACScan was used for flow cytometric analysis of the DNA content of cells stained with propidium iodide (Epstein and Cross, 1992). Clb2p was detected by immunoblotting using a polyclonal rabbit antiserum (Surana, et al., 1993) and an enhanced chemoluminescence detection system (ECL, Amersham). Clb2-associated Cdc28 kinase activity was detected using histone H1 as a substrate as described (Surana, et al., 1993). Kinase activities were quantified with a Molecular Dynamics PhosphorImager. A *clb2* deletion strain (K1890) was used for negative controls and Swi6p was detected as a loading control.

Strains K6208 and K6131 were constructed by replacing the endogenous *CLB2* genes of a wild type and an *apc1-1* strain, respectively, as described in example 4A. A *CLB2-myc12* strain was crossed to a Δ*clb1* strain and tetrads were dissected. Δ*clb1 CLB2-myc12* strains obtained in this cross grew normally at 37°C, demonstrating that the Clb2-myc12 protein was fully functional (In a *clb1* deletion strain, *CLB2* is essential for viablity).

Fig. 5A shows the percentage of budded cells, of cells containing a short spindle in an undivided nucleus, and of cells with separated chromosomes and an elongated spindle (late anaphase-telophase). In *apc1-1* cells budding and the formation of mitotic spindles occured at the same time as in wild type cells but entry into anaphase was delayed by approximately 20 min. Most cells *apc1-1* cells later underwent anaphase but were slow in dissasembling their mitotic spindles. *apc1-1* cells did not undergo cytokinesis.

Fig. 5B shows the distribution of the DNA content. *apc1-1* cells initiated DNA replication at the same time as wild type cells. In contrast to wild type cells the mutant cells arrested with replicated DNA at 37°C.

Fig. 5C shows Clb2 protein and Clb2p-associated kinase activity. In *apc1-1* cells Clb2 protein and Clb2p-associated kinase activity appeared later than in wild type cells. Neither Clb2 protein nor Clb2p-Cdc28p kinase activity declined as *apc1-1* cells underwent anaphase.

Fig. 5D shows defective Clb2p destruction and lack of astral microtubules in *apc1-1* cells. A wild-type (K6208) and an *apc1-1* (K6131) strain, both containing *CLB2-myc12*, were grown for two days at 25°C on YEP-2% glucose-plates to obtain unbudded G1 cells. Strains were inoculated into liquid medium (YEP 2% glucose) at 37°C and samples for flow cytometric analysis of cellular DNA content and indirect immunofluoreseence were taken at one-hour intervals. DNA replication and the formation of buds occured at the same time in wild type and *apc1-1* cells. Clb2-myc12p was detected with the 9E10 antibody and a CY3-conjugated secondary antibody. Spindles were detected with a rabbit antiserum to yeast tubulin and a FITC-conjugated secondary antibody. Pictures were taken with a CCD camera mounted on a Zeiss Axiophot microscope. Fig. 5D shows immunofluorescence pictures from samples taken after 3 hours. In *apc1-1* cells, Clb2-myc12p is present in high amounts in cells with fully elongated spindles (cells that underwent anaphase) whereas Clb2-myc12p is degraded during anaphase in wild type cells. *apc1-1 c*ells are defective in the formation of astral microtubules emanating from the poles of mitotic spindles.

### REFERENCES

A. W. Murray, M. J. Solomon, M. W. Kirschner, *Nature* **339**, 503 (1989); F. C. Luca, E. K. Shibuya, C.E. Dohrman, J.V. Ruderman, *EMBO J*. **10**, 4311 (1991).
U. Surana *et al*., *EMBO J*. **12**, 1969 (1993).
C. Dahman, J. F. X. Diffley, K. Nasmyth, *Curr. Biol*. **5**, 1257 (1995).
R. W. King *et al*., *Cell* **81**, 279 (1995).
V. Sudakin *et al*., *Mol. Biol. Cell* **6**,185 (1995).
A. Ciechanover, *Cell* **79**, 13 (1994); J. M. Peters, *Trends Biochem. Sci*. **19**, 377 (1994).
S. Irniger, S Piatti, C. Michaelis, K. Nasmyth, *Cell* **81**, 269 (1995).
W. Zachariae and K. Nasmyth, *Mol. Biol. Cell* **7**, 791 (1996).
T. Hirano, Y. Hiraoka, M. Yanagida, *J. Cell Biol*. **106**,1171 (1988);
K. L. O'Donnell, A. H. Osmani, S. A. Osmani, N. R. Morris, *J. Cell Sci*. **99**, 711 (1991)
I. Samejima and M. Yanagida, *J. Cell Biol*. **127**, 1655 (1994)
S. Tugendreich, J. Tomkiel, W. Earnshaw, P. Hieter, *Cell* **81**, 261 (1995).
J. R. Lamb, W. A. Michaud, R. S. Sikorski, P. A. Hieter, *EMBO J*. **13**, 4321 (1994).
S. L. Holloway, M. Glotzer, R. W. King, A. W. Murray, *Cell* **73**,1393 (1993).
H. Funabiki *et al*., *Nature* **381**, 438 (1996).
A. Amon, S. Irniger, K. Nasmyth, *Cell* **77**, 1037 (1994).
H. Akari, K. Awane, N. Ogawa, Y. Oshima, *Mol. Gen. Genet*. **231**, 329 (1992).
D. B. Engle *et al.*, *J. Biol. Chem*. **265**, 16132 (1990).
M. Starborg, E. Brundell, K. Gell, C. Höög, *J. Biol. Chem*. **269**, 24133 (1994).
J. M. Peters, R. W. King, C. Höög, M. W. Kirschner (1996), submitted.
S. A. Osmani, D. B. Engle, J. H. Doonan, N. R. Morris, *Cell* **52**, 241 (1988).
S. W. James, P. M. Mirabito, P. C. Scacheri, N. R. Morris, *J. Cell Sci.* **108**, 3485 (1995).
F. Cvrckova and K. Nasmyth, *EMBO J*. **12**, 5277-5286 (1993)
F. Sherman, *Methods Enzymol*. **194**, 3 (1991)
K. Nasmyth, G. Adolf, D. Lydall, A. Seddon, *Cell* **62**, 631 (1990)
E. Schwob and K. Nasmyth, *Genes Dev*. **7**, 1160 (1993)
F. M. Ausubel, et al., *Current Prot in Mol Biol* 1, (1994)
C. B. Epstein and F. R. Cross, *Genes Dev*. **6**, 1695 (1992)
Längle-Rouault and Jacobs, *NAR* **23**, 3079-3081 (1995)
Gietz and Sugino, *Gene* **74**, 527 (1988)
C.B Epstein and F. R. Cross, *Genes Dev*. **6**, 1695 (1992)
Harlow, E. and Lane, D., Antibodies, A Laboratory Manual, Cold Spring Harbor Laboratory (1988)
Sikorski and Hieter, *Genetics* **122**, 19-27 (1989)
Tugendreich et al., *PNAS* **90**, 10031-10035
O'Reilly et al. Baculovirus Expression Vectors. A Laboratory Manual. Ed. Freeman &Co, New York, 1992
Guthrie, C. and Fink, G. R. Guide to Yeast Genetics and Molecular Biology, *Meth. Enzym.* **194** (1991)
Glotzer, M, et al., *Nature* **349**, 132-138 (1991)
Rolfe, et al., *PNAS* **92**, 3264-3268 (1995)
Ellison, J. M. and Hochstrasser, M., *Journ Biol Chem* **266**, 21150-21157 (1991)

## Claims

1. A method for identifying novel subunits of the human Anaphase Promoting Complex (APC), characterized by the steps
a) replacing in a cell of the budding yeast *Saccharomyces cerevisiae* one or more endogenous genes encoding a known APC subunit by epitope-tagged versions of said genes or transforming the cell with a vector containing the corresponding epitope-tagged cDNA(s),
b) growing yeast cells obtained in a) and preparing a protein extract,
c) isolating the yeast APC by contacting the protein extract obtained in b) with an antibody directed against the epitope-tag,
d) isolating the antibody-bound yeast protein(s) and purifying them,
e) determining the sequence of the yeast protein(s),
f) identifying the human subunit(s) by comparing the sequence(s) of the yeast protein(s) obtained in e) and/or the DNA sequence encoding those proteins with published human sequences.

2. The method of claim 1, wherein the gene(s) used in step a) are selected from the group *APC1, CDC16, CDC23, CDC26, CDC27.*

3. A method for identifying novel subunits of the human Anaphase Promoting Complex (APC), characterized by the steps
a) replacing in a human cell one or more endogenous genes encoding known APC subunits by epitope-tagged versions of said genes or transforming the cell with a vector containing the corresponding epitope-tagged cDNA(s) and establishing a cell line,
b) growing the cell line obtained in a) and preparing a protein extract,
c) isolating the APC by contacting the protein extract obtained in b) with an antibody directed against the epitope-tag,
d) isolating the antibody-bound protein(s) and purifying them,
e) determining the sequence of the protein(s).

4. The method of claim 3, wherein the gene(s) or the respective cDNAs used in step a) are selected from a group of genes that are homologs of the yeast genes *APC1, CPC16*, *CDC23, CDC26, CDC27.*

5. A method for producing recombinant APC, characterized in that cDNAs encoding APC subunits are expressed in a suitable host, said subunits are isolated, purified and allowed to assemble to form a functional APC.

6. The method of claim 5, wherein the cDNAs are expressed in a Baculovirus expression system.

7. The use of recombinant APC in a screening method for identifying substances that inhibit rapidly proliferating cells by interfering with the cells' entry into the subsequent cell cycle.

8. The use of claim 7 wherein said method comprises determining the effect of a substance on the APC's ability to ubiquitinate a substrate.

9. The use of claim 7 or 8, wherein the APC and the substrate are of human origin.

10. The use of claim 9, wherein the substrate is a recombinant cyclinB.
